Europäisches Patentamt

European Patent Office

Office européen des brevets

(19)

(11) Publication number: **0 305 432 B1**

(12) **EUROPEAN PATENT SPECIFICATION**

(45) Date of publication of patent specification: **28.10.92**  (51) Int. Cl.⁵: **A61F 5/441**

(21) Application number: **88901901.4**

(22) Date of filing: **01.03.88**

(86) International application number:
**PCT/GB88/00149**

(87) International publication number:
**WO 88/06433 (07.09.88 88/20)**

(54) **A FILTER FOR ATTACHMENT TO AN OSTOMY BAG.**

(30) Priority: **02.03.87 GB 8704826**

(43) Date of publication of application:
**08.03.89 Bulletin 89/10**

(45) Publication of the grant of the patent:
**28.10.92 Bulletin 92/44**

(84) Designated Contracting States:
**AT BE CH DE FR IT LI LU NL SE**

(56) References cited:
**EP-A- 0 210 032**
**GB-A- 2 116 433**
**GB-A- 2 122 090**
**GB-A- 2 177 301**
**US-A- 4 516 974**

(73) Proprietor: **E.R. Squibb & Sons, Inc.**
**Lawrenceville-Princeton Road**
**Princeton, N.J. 08540-4000(US)**

(72) Inventor: **STEER, Peter, Leslie**
**"Kingscote" Woodlands Rise**
**East Grinstead Sussex RH19 1EY(GB)**

(74) Representative: **Cook, Anthony John et al**
**D. YOUNG & CO. 10, Staple Inn**
**London, WC1V 7RD(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to a filter for attachment to an ostomy bag as specified in the preamble of claim 1. Such a filter is known from U.K. Patent No. (GB-A)2 116 433.

There has been proposed, in our U.K. Application Serial No. (GB-A)2 177 926, a filter housing forming part of a bag side element of an ostomy coupling. Within this filter housing is received a disc of a filter medium. One example of a suitable filter element is that disclosed and claimed in our U.K. Patent Application No. (GB-A) 2 171 052. While in many circumstances it is convenient to have a filter housing integral with a bag side coupling element, for certain kinds of ostomy and similar bags for receiving discharged waste material from the human body, it is desirable to have a filter which is particularly adapted to be attached to the wall of an ostomy bag. One method of attachment is the use of an adhesive patch filter and another arrangement is the employment of a fold-over filter, see our U.K. Patent 2 122 090. U.K. Patent Application No. 2 116 433 shows a filter housing attached to a wall of an ostomy pouch. The housing is circular and contains therein a closely-fitting disc of a filtering and deodorising medium. The lid of the housing is hinged on and has a central hole. It also has an annular channel which fits over and co-operates with a circular rib forming part of the base of the housing. Within the pouch, barrier film (13) is disposed within the pouch to provide two chambers in the pouch, the chambers being in communication via vent aperture 20, and to protect the filter element from contact with and possible obstruction by exudate. Interior wall surface areas of the double-chambered pouch are raised or embossed (13a, 13b), and the filter assembly is internally ribbed, to ensure that the pathway for the escape of gases from the pouch remains open. In contrast to these suggestions, the present invention aims to provide an improved bag-filter combination to meet the need for a filter which can be permanently attached to the bag at a chosen position but which nevertheless permits exchanging the filter medium.

According to the present invention, there is provided a filter for attachment to an ostomy bag, the filter comprising three parts, the first part including a synthetic plastics base member having a central aperture therein, there being at least one wall upstanding from the base member and the wall defining with the base member a shallow recess, and the base member having a substantially flat surface whereby the filter can be attached, by plastics welding or adhesive, to a wall of an ostomy bag; the second part being a filter pad consisting essentially of a filtering medium and having lateral dimensions such that it conforms to and fits within the recess, and the third part being a lid member of synthetic plastics material, one of the lid members and the base members including a channel and the other including a wall which is a snug fit within said channel in the closed position of the lid member whereby the filter can be permanently attached to the bag and the lid member is capable of being opened to allow replacement of said pad; the filter pad and base member being both of substantially circular configuration; characterised in that the lid member has a substantially flat lid portion provided with apertures therethrough adjacent to and interiorly of the wall on the lid member, said apertures being restricted to a peripheral array; in that two walls are upstanding from the base member and define a channel, the radially inner wall which defines the said recess and is part of said channel being chamfered at its upper, radially outer, region; in that the wall is on the lid member and in the closed position of the lid member makes a snug fit with the said channel so as to give a secure attachment between the lid member and the base member, said filter pad having a height substantially equal to that of the recess; and in that the the wall on the lid member and/or one or both of the walls on the base member has a projection or a series of projections whereby a snap fit can be achieved between the base and lid members and whereby exchange of the filter medium is easily effected manually by removal and replacement of the lid portion.

The wall on the lid member may have a radially outwardly extending rim so as to enhance its snap fit co-operation with the aforesaid channel.

The invention will be better understood from a consideration of the following description of an illustrative embodiment thereof, given with reference to the accompanying drawings, in which:-

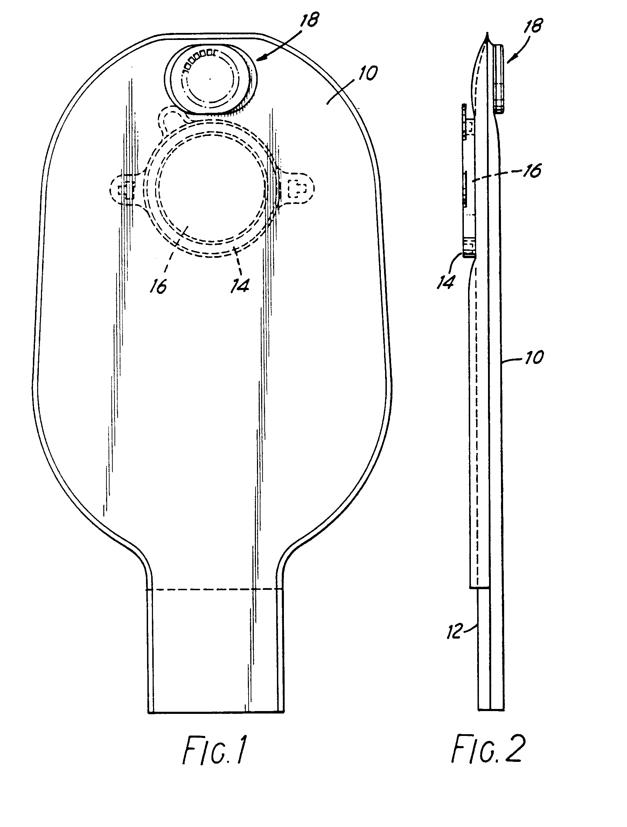

Figure 1 is a front view of an ostomy bag including a filter in accordance with one example of the present invention;

Figure 2 is a diagrammatic side view of the ostomy bag shown in Figure 1;

Figure 3 is a front view of the base member of an example of filter in accordance with the present invention;

Figure 4 is a cross-sectional view on a line 4-4 in Figure 3 of the base member;

Figure 5 and Figure 6 are respectively a front view and a side view of a filter pad for use in a filter according to the present invention;

Figure 7 is a front view of a lid member for use in a filter according to the present invention;

Figure 8 is a rear view of the lid member shown in Figure 7, and

Figure 9 is a cross-sectional view on the lines IX-IX of Figures 7 and 8.

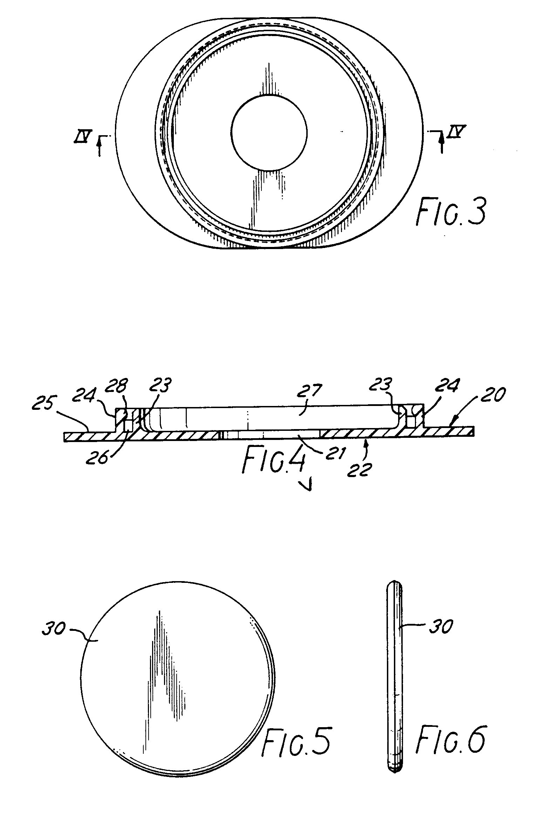

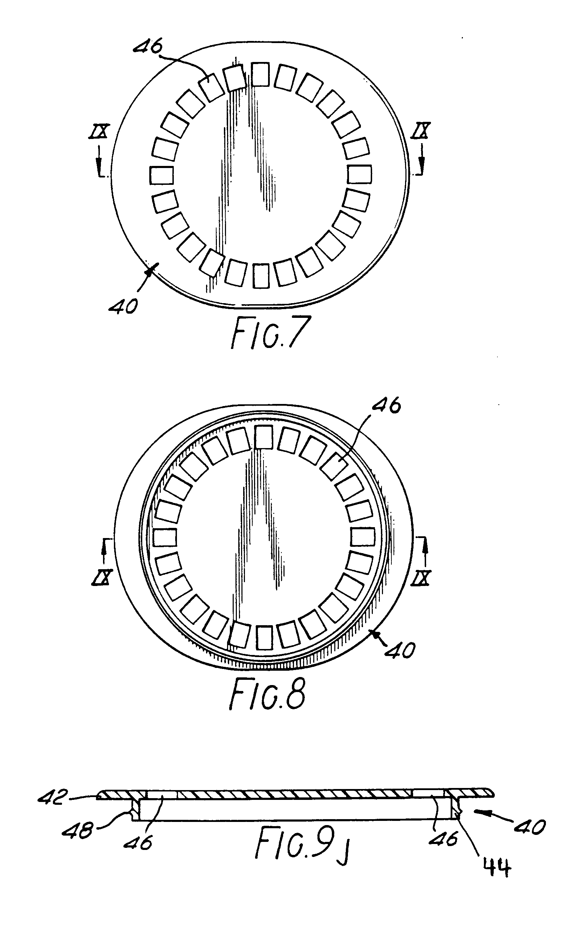

The illustrated ostomy bag has a front wall 10 and a rear wall 12. Front and rear are used in the context of a forward facing person wearing an ostomy bag on a forward part of his abdomen. The rear wall 12 of the ostomy bag carries a bag side ostomy coupling. This is intended to co-operate with a body side coupling in a manner now well known in the art, see for example British Patent 1 571 657. The bag side coupling is shown at 14. It surrounds a stomal aperture 16 in the wall of the bag. The front wall 10 carries a filter 18 in accordance with the present invention. Details of the filter will be better understood from Figures 3-9.

The filter includes a first part or base member 20, a filter pad 30, and a lid member or third part 40.

The base member 20 is of synthetic plastics material and has a central aperture 21 therein and one substantially flat surface 22 whereby the filter can be attached by plastics welding or adhesive to the wall 10 of the ostomy bag.

Two walls 23, 24 are upstanding from the other side 25 of the base member to define a channel 26 therebetween. The inner wall defines with the base member a shallow recess 27. In the illustrated embodiment of the invention, the walls 23 and 24 are generally circular and the recess is of a shallow cylindrical shape. However, the advantages of the invention do not necessarily require a circular configuration for the walls and a cylindrical recess, other shapes may be employed as will be understood by a man of average skill in the art. The outer wall 24 as seen in Figure 4, has an internal peripheral rim or projection 28.

The second part shown in Figures 5 and 6 is a filter pad of suitable material which permits a passage of gases. It may carry activated carbon as filter material. Any known suitable filter pad or disc may be employed but a particularly advantageous one is that disclosed and claimed in our British Patent Application 2 171 052.

The third part of the illustrated filter according to the present invention is the lid member 40, and this has a generally flat lid portion 42 and a cylindrical wall 44 extending therefrom. It also has a series of apertures 46 arranged to provide a plurality of peripherally arranged exit paths for filtered gas to pass to the exterior. These apertures are shown at 46. As illustrated, the wall 44 has a peripheral external rim or projection 48 at its end further from the lid portion 42. The projection 48 is pushed past the projection 28 on the wall 24 when the wall 44 is inserted into the channel 26 as occurs when the lid is placed on the base member. The presence of the projections described gives a secure attachment of the lid to the base member but nevertheless this construction is an arrangement in which an easy manual separation of these two parts can be obtained, e.g. when it is desired to change the filter disc in the recess 27.

## Claims

1. A filter for attachment to an ostomy bag, the filter comprising three parts, the first part including a synthetic plastics base member (20) having a central aperture (21) therein, there being at least one wall upstanding from the base member and the wall defining with the base member a shallow recess (27), and the base member (20) having a substantially flat surface (22) whereby the filter can be attached, by plastics welding or adhesive, to a wall of an ostomy bag; the second part being a filter pad (30) consisting essentially of a filtering medium and having lateral dimensions such that it conforms to and fits within the recess, and the third part being a lid member (40) of synthetic plastics material, one of the lid members and the base members including a channel and the other including a wall (44) which is a snug fit within said channel in the closed position of the lid member whereby the filter can be permanently attached to the bag and the lid member is capable of being opened to allow replacement of said pad; the filter pad (30) and base member (20) being both of substantially circular configuration; characterised in that the lid member (40) has a substantially flat lid portion (42) provided with apertures (46) therethrough adjacent to and interiorly of the wall (44) on the lid member, said apertures being restricted to a peripheral array; in that two walls (23, 24) are upstanding from the base member (20) and define a channel, the radially inner wall (23) which defines the said recess (27) and is part of said channel being chamfered at its upper, radially outer, region; in that the wall (44) is on the lid member and in the closed position of the lid member makes a snug fit with the said channel so as to give a secure attachment between the lid member (40) and the base member (20), said filter pad (30) having a height substantially equal to that of the recess; and in that the the wall (44) on the lid member and/or one or both of the walls (23,24) on the base member has a projection (48 and/or 28) or a series of projections whereby a snap fit can be achieved between the base and lid members and whereby exchange of the filter medium is easily effected manually by removal and replacement of the lid portion.

## Patentansprüche

1. Filter zur Anbringung an einem Ostomiebeutel, wobei der Filter drei Teile aufweist, das erste Teil ein Kunststoff-Basiselement (20) mit einer Zentralöffnung (21) darin aufweist, wobei mindestens eine Wand, die von dem Basiselement absteht, vorhanden ist, und diese Wand mit dem Basiselement eine flache Ausnehmung (27) bildet und das Basiselement (20) eine im wesentlichen flache Fläche (22) hat, wobei der Filter mittels Kunststoffschweißen oder Klebstoff an einer Wand eines Ostomiebeutels angebracht werden kann, das zweite Teil ein Filterkissen (30) ist, das im wesentlichen aus einem Filtermedium besteht und solche äußeren Abmessungen hat, daß es mit der Ausnehmung übereinstimmt und in diese hineinpaßt, und das dritte Teil ein Deckelelement (40) aus Kunststoffmaterial ist, wobei entweder das Deckelelement oder das Basiselement einen Kanal aufweist und das jweils andere Element eine Wand (44) aufweist, die in der geschlossenen Position des Deckelelements genau in den Kanal hineinpaßt, wodurch der Filter dauerhaft an dem Beutel angebracht werden kann und das Deckelelement zum Austausch des Kissens geöffnet werden kann, das Filterkissen (30) und das Basiselement (20) beide eine im wesentlichen kreisförmige Ausbildung haben, **dadurch gekennzeichnet**, daß das Deckelelement (40) einen im wesentlichen flachen Deckelabschnitt (42) hat, der benachbart zu und innenliegend von der Wand (44) auf dem Deckelelement mit durchgehenden Öffnungen (46) versehen ist, wobei die Öffnungen auf einen äußeren Bereich beschränkt sind, und daß zwei Wände (23, 24) von dem Basiselement (20) abstehen und einen Kanal bilden, wobei die radial gesehen innere Wand (23), die die Ausnehmung (27) bildet und ein Teil des Kanals ist, an ihrem oberen radial gesehen, äußeren Bereich abgeschrägt ist, daß die Wand (44) an dem Deckelelement ist und in der geschlossenen Position des Deckelelements mit dem Kanal einen genauen Sitz mit dem genannten Kanal bildet, so daß eine sichere Anbringung zwischen dem Deckelelement (40) und dem Basiselement (20) bereitgestellt wird, wobei das Filterkissen (30) eine Höhe im wesentlichen gleich der der Ausnehmung hat, und daß die Wand (44) an dem Deckelelement und/oder eine oder beide der Wände (23, 24) an dem Basiselement einen Vorsprung (48) und/oder (28) oder eine Serie von Vorsprüngen haben, wodurch ein Schnappsitz zwischen dem Basis- und Deckelelement erreicht werden kann und wodurch ein Wechsel des Filtermediums durch Abnehmen und Austausch des Deckelabschnitts leicht von Hand durchgeführt werden kann.

## Revendications

1. Filtre à fixer à un sac de stomie, ce filtre comprenant trois parties, la première partie comportant une base (20) en matière plastique synthétique, dans laquelle est pratiquée une ouverture centrale (21), au moins une paroi s'élevant de la base et cette paroi délimitant avec la base un évidement peu profond (27), et la base (20) ayant une surface sensiblement plate (22) par laquelle le filtre peut être fixé, par soudage plastique ou par adhésif, à une paroi d'un sac de stomie; la seconde partie consistant en un tampon filtrant (30) composé essentiellement d'un milieu filtrant et dont les dimensions latérales sont telles qu'il épouse l'évidement et s'y adapte, et la troisième partie consistant en un couvercle (40) en matière plastique synthétique, l'un ou l'autre du couvercle et de la base comportant une gorge et l'autre comportant une paroi (44) qui s'encliquète dans ladite gorge, dans la position fermée du couvercle, grâce à quoi le filtre peut être fixé à demeure au sac et le couvercle peut être ouvert pour permettre le remplacement dudit tampon; le tampon filtrant (30) et la base (20) étant tous deux de forme sensiblement circulaire; caractérisé en ce que le couvercle (40) a une partie sensiblement plate (42) percée d'ouvertures (46) à proximité de et intérieurement à la paroi (44) du couvercle, lesdites ouvertures étant restreintes à une rangée périphérique; en ce que deux parois (23,24) s'élèvent de la base (20) et délimitent une gorge, la paroi radialement intérieure (23) qui délimite ledit évidement (27) et fait partie de ladite gorge étant chanfreinée dans sa région supérieure et radialement extérieure; en ce que la paroi (44) se trouve sur le couvercle et, dans la position fermée du couvercle, est encliquetée dans ladite gorge de manière à assurer une fixation sûre entre le couvercle (40) et la base (20), ledit tampon filtrant (30) ayant une hauteur sensiblement égale à celle de l'évidement; et en ce que la paroi (44) du couvercle et/ou une ou les deux parois (23,24) de la base comporte(nt) une partie saillante (48 et/ou 28) ou une série de parties saillantes pour qu'un encliquetage puisse être réalisé entre la base et le couvercle et pour que l'échange du milieu filtrant se fasse aisément à la main par enlèvement et remplacement de ladite partie du couvercle.

FIG.1

FIG.2

FIG.3

FIG.4

FIG.5

FIG.6

FIG.7

FIG.8

FIG.9